# EUROPEAN PATENT APPLICATION

(11) **EP 2 567 720 A1**
(43) Date of publication of application: **13.03.2013**
(21) Application number: 12179488.7
(22) Date of filing: 07.08.2012
(51) Int. Cl.: A61M 5/165, A61M 5/14, A61M 5/38

(54) **Medical infusion filter with optimized filling**

(30) Priority: 08.09.2011 IT MI20111621
(71) Applicant: GVS S.p.A., 40069 Zola Predosa (Bologna) (IT)
(72) Inventor: Scagliarini, Massimo, 40033 Casalecchio Di Reno (Bologna) (IT)
(74) Representative: Ripamonti, Enrico

(57) **Abstract**

A medical infusion filter (1) comprises a box body (2) defined by at least two half shells (3, 4) coupled together to define an internal cavity (7) divided into at least two chambers (8, 9) by at least one hydrophilic filtering membrane (5), these chambers comprising at least one first or entry chamber (8) and at least one second or exit chamber (9), the at least one first or entry chamber (8) being connected to an entry conduit (10) for the medical fluid originating from a corresponding container and the at least one second or exit chamber (9) being connected to an exit conduit (11), at least the first or entry chamber (8) provided within a first half shell (3) presenting at least one aperture (13, 14) within which a corresponding hydrophobic membrane (16, 17) is positioned. The at least one aperture (13, 14) enables a differential air flow rate to pass towards the exterior of said box casing (2) as a function of the distance of the casing air exit zone from the entry conduit.

## Description

The present invention relates to a filter in accordance with the introduction to the main claim.

Filters used in medical infusion lines to filter a fluid directed to a patient have been known for some time, for example to extract agents therefrom which are potentially infective for the patient and/or to eliminate from the fluid any air bubbles which if introduced into a patient's vein cold cause serious damage thereto. In particular, filters of the aforesaid type are known for administering drugs to a patient by devices provided with a pumping element.

All these filters generally comprise a box body defined by at least two half shells coupled together to define at least one internal cavity. Each cavity is preferably divided into at least two chambers by a hydrophilic filtering membrane. Such filters also comprise a conduit for entry of the medical fluid (to be connected to a fluid feed line emerging from a container or vessel of this fluid) connected to at least one first chamber for entry to said cavity; said filters also comprise an outlet conduit (to be connected to a tube for directing the fluid to a patient after filtering) connected to at least one second chamber for exit from said cavity of the box casing.

In order to operate this filtration, each entry and exit chamber pair presents a hydrophilic membrane which separates them and through which the fluid passes, but not any air contained therein, before entering the tube connected to the patient. The air in this fluid is usually blocked in the first chamber and is expelled through one or more apertures provided in the first shell on which corresponding hydrophobic membranes are positioned.

In such a filter it is of fundamental importance to carry out priming when connected to the medical fluid container. This initial priming procedure or step enables the air (in the form of bubbles or microbubbles) present in its interior to be eliminated before directing the medical fluid to the patient (by connecting the filter outlet tube to the patient by known methods), to prevent air reaching the patient's vein.

This procedure must be carried out carefully, but sufficiently rapidly to be able to proceed with the infusion of the medical fluid to the patient without succumbing to the high risk that the air present in the second filter chamber has not been completely extracted. This latter may also not be detected on commencing infusion, with obvious possible drawbacks during this latter procedure. Moreover, precisely to enable the air to exit the second chamber, the priming procedure on a generic filter must be carried out while maintaining this latter in a correct spatial arrangement which facilitates air outflow from the exit conduit of the filter casing. This operation hence requires care by a health operative or by the filter user.

An object of the present invention is to provide a medical infusion filter which represents an improvement on known filters.

A particular object of the invention is to provide a filter of the stated type which offers optimized filling and, on priming, enables all air contained in the second chamber to be securely removed.

Another object is to provide a filter of the stated type which is reliable in use and can be produced by standard methods, i.e. similar to those used for producing known filters.

A further object is to provide a filter of the stated type which enables priming to be carried out without the need for a particular spatial arrangement.

These and other objects which will be apparent to the expert of the art are attained by a filter in accordance with the accompanying claims.

The present invention will be more apparent from the accompanying drawings, which are provided by way of non-limiting example and in which:

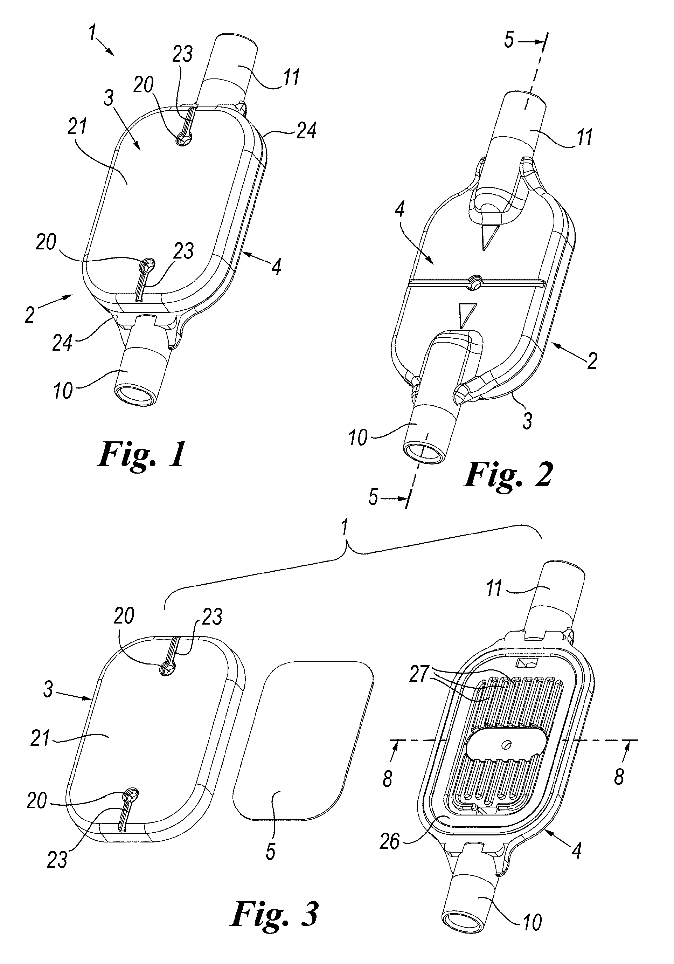

Figure 1 is a perspective view of a filter according to the invention seen from one side;
Figure 2 is a perspective view of the filter of Figure 1 seen from another side;
Figure 3 is an exploded perspective view of the filter shown in Figure 1;

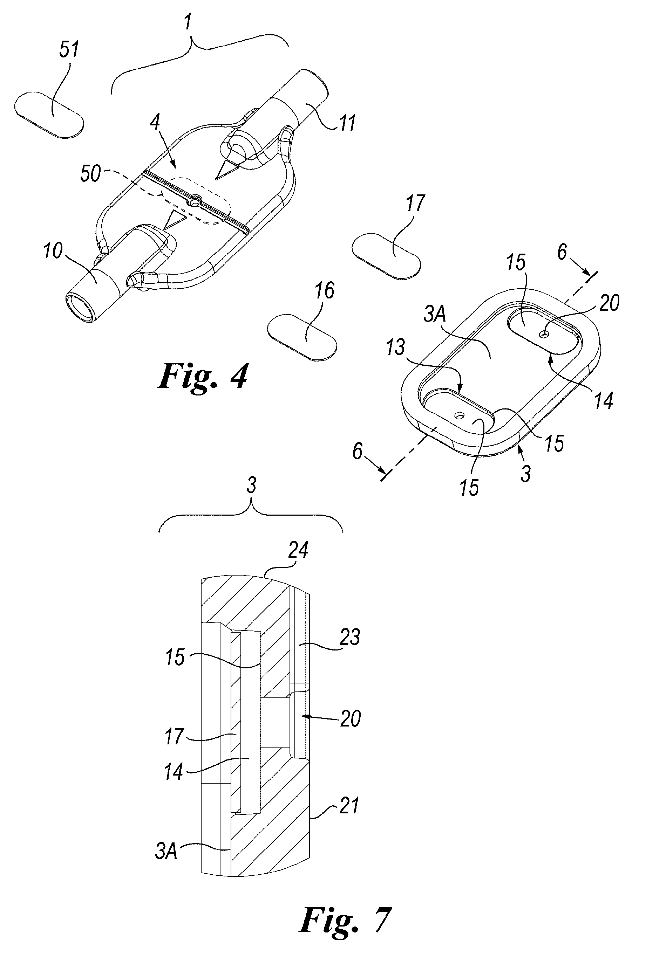

Figure 4 is an exploded perspective view of the filter shown in Figure 2;

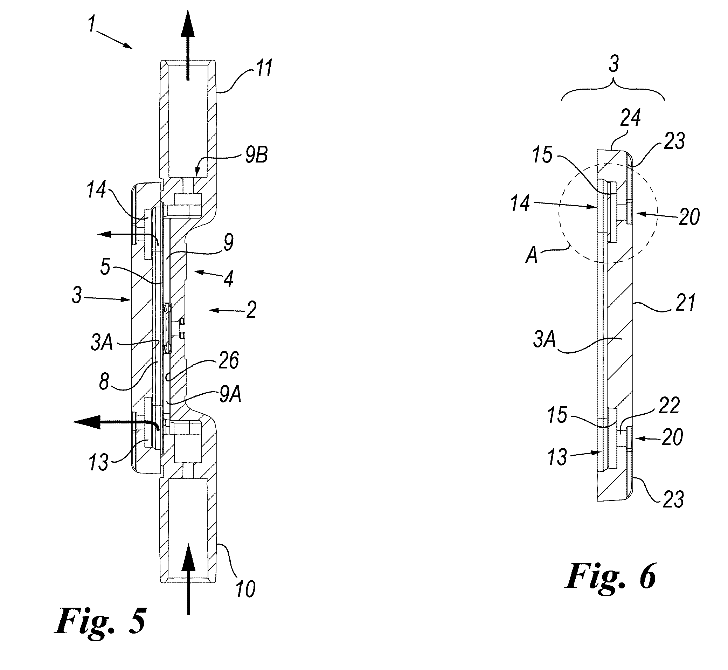

Figure 5 is a schematic section on the line 5-5 of Figure 2;
Figure 6 is a schematic section on the line 6-6 of Figure 4;
Figure 7 is an enlarged view of the part indicated by A in Figure 6; and
Figure 8 is an enlarged cross-sectional view of a part of the filter according to the invention.

With reference to said figures, an example of a filter according to the invention is indicated overall by 1 and comprises a box casing 2 defined, in the illustrated embodiment (as stated, provided by way of non-limiting example and to which the present description of inventive embodiments refers) by a first and a second element or half shell 3, 4 enclosing an intermediate element 5. These box casing elements 3, 4 are formed preferably of plastic material in any known manner, whereas the intermediate element is a known hydrophilic membrane. The half shells 3 and 4 are coupled together in any known manner (for example by thermo-welding or ultrasound or other known methods) and define within the casing 2 an internal cavity 7 divided by the membrane or intermediate element 5 into two chambers 8 and 9.

The first chamber 8 (or entry chamber) is connected to a conduit 10 for the entry of a medical fluid into the casing 2; this conduit is to be connected to a tube (not shown) for feeding the medical fluid (originating from a container such as a bag or the like, not shown) to the filter 1. The second chamber 9 (or exit chamber) is connected to a conduit 11 for the exit of said fluid, after its filtration through the hydrophilic membrane 5; this conduit 11 is arranged to be connected to a tube (not shown) for feeding the fluid to a patient.

Both the conduit 10 and the conduit 11 form part of the casing 2 of the filter 1. Said cavity can be divided by several membranes into several chambers 8 and 9, at least one of which represents a first chamber for entry and at least one of which represents a second chamber for exit.

In the embodiment of the figures, the first half shell 3 comprises two separate spaced-apart apertures 13 and 14, a first 13 being close to the entry conduit 10. Thee apertures present, on that said 3A of the half shell 3 facing the first chamber 8, a seal (elongated in the example) 15 arranged to contain a corresponding known hydrophobic membrane 16, 17. Each seat 15 is connected to a through hole 20 opening on the outer side 21 (with respect to the internal cavity 7) of the half shell and connected to a recess 23 lying axially on the casing 2 and terminating at an edge 24 of the half shell 3.

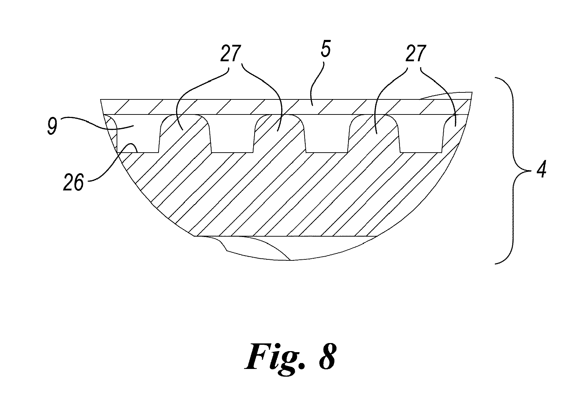

The second half shell 4 presents an inner side 26 (facing the second chamber 9) from which a plurality of ribs 27 upwardly extend to cooperate with the hydrophilic membrane 5 and to support this latter. These ribs can have a free end 27A which is flat (as in Figure 8) or rounded. It should be noted that the shape and proportion of the ribs, their depth and the distance between the half shells 3 and 4 are factors which affect the correct operation of the filter.

The (ventilation) apertures 13 and 14 of the first half shell, which contain the corresponding membranes 16, 17, enable air to flow from the inside of the casing 2 to the outside with mutually different flow rates.

In particular, the air flow rate which can pass through the aperture 13 is greater than that which can pass through the aperture 14.

By virtue of this characteristic, during the filter priming step, optimal and complete emptying of the air of the second chamber 9 is achieved. In this respect, on connecting the entry conduit 10 to a container of medical fluid, this latter begins to penetrate into the first chamber 8 and the air present therein flows out mainly through the aperture 13 (and the corresponding membrane 16), whereas it flows out with much greater difficulty through the other aperture 14. In this manner, at this latter aperture a sort of air pocket (or overpressure) develops in the chamber 8 and presses on the entry fluid, to maintain it close to the conduit 10 and force it to pass through the hydrophilic membrane 5 even at that portion of this latter close to the conduit 10. It follows that the fluid begins to penetrate into the second chamber 9 at that portion of this latter most distant from the exit conduit 11. Following this, the air present in the chamber 9 begins to be forced towards the conduit 11 starting from that portion of this chamber most distant from this latter; as the medical fluid penetrates into the chamber 9 in the aforesaid manner, the air present in this latter discharges to the outside through the exit conduit 11, this outflow taking place subsequent to the filling of this chamber always from the fluid-free portion most distant from said conduit. Consequently, in contrast to already known filters, the present invention presents presser means (the apertures 13 and 14 with different air flow rates) which enable a suitable overpressure to be created in the chamber 8 such as to urge the fluid to pass through the membrane 5. In its turn, this fluid, after passing into that free zone of the chamber 9 most distant from the exit conduit 11, urges the air present therein towards this conduit, hence completely emptying this chamber.

At the same time, the fluid also fills the first chamber 8 to press the air present therein out through the apertures 13 and 14.

The aforedescribed continues until the two chambers are completely filled with medical fluid and the air has been totally expelled from the casing 2 of the filter 1. This latter can hence be connected to the tube feeding the medical fluid to the patient.

The flow rate difference between the two apertures 13 and 14 can be achieved in various ways. In a first method the hydrophobic membranes 16 and 17 are formed with different filtering surface areas.

The result is a different air flow per time unit through the membrane 16 and relative aperture 13 compared with the flow per time unit through the membrane 17 and aperture 14.

According to a variant this flow rate difference through the apertures 13 and 14 can be achieved by modifying the relative porosity of the membranes 16 and 17.

The flow difference can also be obtained by using membranes 16 and 17 formed of suitable but different materials such as to achieve said difference.

According to a further variant, the membranes 16 and 17 can be of identical material but present different thicknesses or cross-sections (even obtained by superimposing a different number of flat layers); again in this case a different air exit flow rate is obtained from the corresponding apertures 13 and 14.

Further variants can be obtained by at least one of the following solutions, which comprise:
- a difference in the shape of the two membranes 16 and 17 including, but not only, in the presence of a difference in the apertures 13 and 14;
- a different positioning of the apertures 13 and 14 and of the membranes 16 and 17 either relative to each other and/or on the half shell, but also on the cover which comprises them. For example, these apertures can be more or less close together or have different positions and/or be displaced about a middle longitudinal axis of the shell 3;
- forming at least one ventilation aperture (indicated by 50 in the figures), provided with its own hydrophobic membrane 51 on that half shell 4 presenting the exit conduit 11 which, by acting in synergy with at least one of the already described solutions, contributes to correct priming of the filter 1;
- modifying the ratio between the surfaces of the hydrophobic membranes 16 and 17 (and relative seats 13 and 14) and the surface of the hydrophobic membrane 5;
- modifying the position of the hydrophobic membranes to the position of the hydrophilic membranes, with the membrane 16 closer to the membrane 5 than the membrane 17 (for example by different membrane thicknesses or a wedge-shaped pattern of the shell 3 on the shell 4).

A further variant also comprises a single aperture provided in the box casing 3, but having a configuration such as to enable optimized air flow. One shape of this type can be triangular or trapezium shaped with very long sides. Alternatively, said single aperture can contain a hydrophobic membrane with differential air permeability which is greater in that part thereof closest to the entry conduit 10.

According to a further variant, as an alternative or in addition to the presser means for the medical fluid which slow down its entry into the chamber 8 of the casing 2 (i.e. the aforesaid apertures and/or the respective membranes), presser means for this fluid can be provided in the exit chamber 9 which force it into that part or zone 9A of this latter which is most distant from the exit conduit 11. These means can be a constriction 9B formed in the half shell 4, in proximity to the exit conduit 11.

The constriction (or diameter reduction of the conduit 11) makes it more difficult for the air to exit the chamber 9; as the liquid which filters through the membrane 5 penetrates into it, this liquid is forced towards that zone 9A of the chamber 9 which is most distant from the conduit 11 because of the pressure of the air which collects in proximity to the constriction 9B or to a reduced diameter zone of this conduit (but within the chamber 9). This pressure forces said liquid towards the zone 9A even if the liquid enters the chamber 9 from any position of this latter, even distant from said zone 9A. In this manner, the air present thereat rises in any event towards the exit conduit to free said chamber. According to another variant, a constriction similar to the aforedescribed constriction 9B (and additional to it) can be provided in proximity to the entry conduit 10. These constrictions can have different cross-sections to hence define, by their ratio, a further presser means which enables the entering the exit chamber to be forced towards the zone 9A of this latter.

Other solutions can be provided, such as that of reducing the cross-section of the aperture 14 compared with the aperture 13; this solution can be obtained from the preceding description, hence falling within the scope of the accompanying claims.

It should be noted that the invention can be applied to any medical infusion filter, both in the case of infusions which take place with very low flow rates, and in the case of administration of drugs for example for oncological therapy, or for parenteral nutrition.

## Claims

1. A medical infusion filter (1) comprising a box body (2) defined by at least two half shells (3, 4) coupled together to define at least one internal cavity (7) divided into at least two chambers (8, 9) by at least one hydrophilic filtering membrane (5), said at least two chambers comprising at least one first chamber (8) and at least one second chamber (9), at least one first chamber or entry chamber (8) being connected to an entry conduit (10) for the medical fluid originating from a corresponding container, and said at least one second chamber or exit chamber (9) being connected to an exit conduit (11), to be connected to a tube for directing the fluid to a patient, the at least one first entry chamber (8) being provided within a first half shell (3) presenting at least one aperture (13, 14) within which a corresponding hydrophobic membrane (16, 17) is positioned and which communicates with the filter exterior, said filter being subjected to a first priming procedure prior to its use, the filter being **characterised by** comprising overpressure means present in at least one from said at least one entry chamber (8) and said at least one exit chamber (9), during the priming procedure said overpressure means forcing the medical fluid entering the box casing (2) from the entry conduit (10) to fill said exit chamber starting from that zone (9A) thereof which is most distant from said exit conduit (11), the filling of the exit chamber (9) achieved in this manner resulting in the exit therefrom of the air present therein when said filter priming procedure is implemented.

2. A filter as claimed in claim 1, **characterised in that** said overpressure means are defined by the at least one aperture provided in the first half shell (3), said aperture having zones which enable differential air flow rates to pass through it.

3. A filter as claimed in claim 2, **characterised in that** the at least one aperture comprises at least one of the following characteristics:
- it has a shape which widens in proximity to the entry conduit (10);
- it contains a hydrophobic membrane having differential air permeability zones, the zone closest to the entry conduit (10) having greater permeability than the zone most distant from the conduit (10).

4. A filter as claimed in claim 1, **characterised in that** the at least one first or entry chamber (8) comprises, formed in the respective half shell, at least two spaced-apart apertures (13, 14) within which corresponding hydrophobic membranes (16, 17) are positioned, such apertures enabling different air flow rates to pass from the at least one first or entry chamber (8) towards the exterior of said box casing, that aperture (13) closest to the fluid entry conduit (10) enable a greater flow rate to pass than that passing through the second aperture (14).

5. A filter as claimed in claim 4, **characterised in that** the membranes (16, 17) of the spaced-apart apertures (13, 14) have at least one of the following mutually different physical characteristics:
- the membrane (16) of that aperture (13) closer to the entry conduit (10) has a cross-section which is smaller than the cross-section of the membrane (17) of the other aperture (14), these membranes having equal surfaces facing the first chamber (8) of the filter casing (2);
- the membrane (16) of that aperture (13) closer to the entry conduit (10) has a filtration area greater than that of the membrane (17) of the other aperture (14);
- the membrane (16) of that aperture (13) closer to the entry conduit (10) has a greater porosity than the membrane (17) of the other aperture;
- the membrane (16) of that aperture (13) closer to the entry conduit (10) is formed of a hydrophobic material which is different from that of the membrane (17) of the aperture (14) more distant from the conduit (10) and with greater permeability than this latter.

6. A filter as claimed in claim 1, **characterised in that** that aperture (14) more distant from the entry conduit (10) has a cross-section less than that of that aperture (13) closer to the conduit (10).

7. A filter as claimed in claim 4, **characterised in that** the apertures (13, 14) and the respective hydrophobic membranes (16, 17) have mutual positions such as to facilitate differential air exit from the chamber to which they pertain.

8. A filter as claimed in claim 4, **characterised by** comprising at least one of the following characteristics:
- the hydrophobic membranes (16, 17) of the at least one first or entry chamber (8) are disposed in positions which are different from the hydrophilic membrane (5);
- the hydrophobic membranes (16, 17) of the at least one first or entry chamber (8) have mutually different areas in relation to the area of the hydrophilic membrane (5).

9. A filter as claimed in claim 4, **characterised by** comprising at least one ventilation aperture (50) in a position corresponding with the at least one second or exit chamber (9) provided in the respective half shell.

10. A filter as claimed in claim 1, **characterised in that** the overpressure means are at least one reduced diameter zone (9B) provided in the second half shell (4) in proximity to the exit conduit (11).
